(19) Europäisches Patentamt
European Patent Office
Office européen des brevets

(11) Publication number: 0 251 186 B1

(12) **EUROPEAN PATENT SPECIFICATION**

(45) Date of publication of patent specification: **01.09.93**

(51) Int. Cl.$^5$: **C07K 15/00**, C12N 5/00, C12N 15/00, C12P 21/00, G01N 33/543, G01N 33/68, G01N 33/577, //(C12P21/00, C12R1:91)

(21) Application number: **87109091.6**

(22) Date of filing: **24.06.87**

# (54) Monoclonal antibody to human plasminogen, method for production thereof, assay reagent and kit comprising said antibody, and hybridoma producing said antibody.

(30) Priority: **24.06.86 JP 146252/86**
**03.12.86 JP 286790/86**

(43) Date of publication of application:
**07.01.88 Bulletin 88/01**

(45) Publication of the grant of the patent:
**01.09.93 Bulletin 93/35**

(84) Designated Contracting States:
**BE CH DE FR GB LI SE**

(56) References cited:

**THE JOURNAL OF BIOLOGICAL CHEMISTRY, vol. 260, no. 22, 5th October 1985, pages 12106-12111, The American Society of Biological Chemists Inc., Washington, D.C., US; P. Holvoet et al.: "A monoclonal antibody specific for Lys-plasminogen"**

(73) Proprietor: **TEIJIN LIMITED**
**11 Minami Honmachi 1-chome Higashi-ku**
**Osaka-shi Osaka-fu(JP)**

(72) Inventor: **Koike, Yukiya**
**First-manshon 102 399-1, Miya**
**Hino-shi Tokyo(JP)**
Inventor: **Wakabayashi, Kenji**
**Teijin Musashino-ryo, 5-18 Tamadaira**
**3-chome**
**Hino-shi Tokyo(JP)**
Inventor: **Sumi, Yoshihiko**
**Teijin Tama-Apart. 113 18-4 Tamadaira**
**3-chome**
**Hino-shi Tokyo(JP)**
Inventor: **Ichikawa, Yataro**
**11-7, Kotesashi-cho, 2-chome**
**Tokorozawa-shi Saitama-ken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACS, vol. 102, 1985, page 390, abstract no. 76465a, Columbus, Ohio, US; H.S. CUMMINGS et al.: "A monoclonal antibody of the epsilon-aminocaproic acid binding site on the kringle 4 region of human plasminogen that accelerates the activation of Glu1-plasminogen by urokinase", & ARCH. BIOCHEM. BIOPHYS. 1985, 236(2), 612-18

CHEMICAL ABSTRACTS, vol. 101, 1984, page 280, abstract no. 68331t, Columbus, Ohio, US; H.S. CUMMINGS et al.: "A probe of the human plasmin-alpha2-macroglobulin interaction utilizing monoclonal antibodies to human plasmin", & ANN. N.Y. ACAD. SCI. 1983, 421, 143-8

BIOLOGICAL ABSTRACTS, vol. 84, 1987, abstract no. 73533; T.K. PLATONOVA et al.: "The effect of endogenous fibrinolysis activation on human plasminogen"

CHEMICAL ABSTRACTS, vol. 100, 1984, page 251, abstract no. 205660v, Columbus, Ohio, US; V.A. PLOPLIS et al.: "Preparation and isolation of monoclonal antibodies to human plasminogen", & PROG. FIBRINOLYSIS 1983, 6, 333-7

CHEMICAL ABSTRACTS, vol. 98, 1983, page 508, abstract no. 32713z, Columbus, Ohio, US; A.V. PLOPLIS et al.: "Monoclonal antibodies to discrete regions of human Glu1-plasminogen", & BIOCHEMISTRY 1982, 21(23), 5891-7

(74) Representative: **Kraus, Walter, Dr. et al**
**Patentanwälte Kraus, Weisert & Partner**
**Thomas-Wimmer-Ring 15**
**D-80539 München (DE)**

## Description

This invention relates to a monoclonal antibody to human plasminogen, a method for its production, an assay reagent and kit, and a hybridoma. More specifically, it relates to a monoclonal antibody which binds specifically to human plasminogen and does not substantially bind to plasmin, a method for production thereof, an assay reagent and kit, and a hybridoma.

Plasminogen is the only digestive protease precursor existing in plasma, and its active form, plasmin, acts on and dissolves fibrin clots resulting mainly from a coagulation reaction. This phenomenon is called fibrinolysis, and plasmin is a main factor involved in the fibrinolysis mechanism.

Plasminogen is synthesized in the liver, and its content in the blood is known to be as high as 150 to 200 micrograms/ml. It can be separated and purified as a single component almost quantitatively by a two-stage operation of affinity chromatography on a Lys-Sepharose column and DEAE ion-exchange column chromatography. Human plasminogen is a single-chain glycoprotein composed of about 800 amino acid residues in total and having a molecular weight of about 90,000 to about 94,000. Glu-plasminogen having a glutamic acid residue at the $NH_2$ terminal and Lys-plasminogen having a lysine residue at the $NH_2$ terminal are known as the human plasminogen. The former is intact plasminogen, and the latter results from cleavage of the Lys-Lys linkage in the $NH_2$ terminal region of the Glu-plasminogen by plasmin. Hence, the molecular weight of Lys-plasminogen is lower than the intact plasminogen by about 8,000.

On the other hand, plasminogen is converted to double-chain plasmin by cleavage of the Arg-Val linkage between the 560th Arg and 561st Val by a tissue activator secreted by various cells (e.g., melanoma, vascular endothelial cells: or urokinase. This plasmin is composed of two chains, a heavy chain (H chain) having a higher molecular weight on the $NH_2$ terminal side and a light chain (L chain) having a lower molecular weight on the COOH terminal side. Two S-S linkages exist between the H and L chains. It is known that a site which plays an important role in enzymatic activity is present in the L chain. Five domains (each consisting of about 80 residues) very similar to each other in amino acid sequence exist in the H chain in addition to sugar chains. These five domains are generally called kringle domains. About 34% of homologous sequences have been found among the individual domains. The lysine binding sites (LBS) in the kringle domains function to interact with fibrin or $\alpha_2$-plasmin inhibitor and are important. It is known that epsilon-aminocaproic acid reacts with LBS to change the higher-order structure of plasmin and affect its reaction with fibrin or $\alpha_2$-plasmin inhibitor [see E. Suenson & S. Thorsen: Biochem. J., 197, 610-628 (1981)].

The Journal of Biological Chemistry, vol. 260, No. 22, pages 12106-12111 (1985) discloses a monoclonal antibody specific for Lys-plasminogen. This article shows that this monoclonal antibody also binds to Lys-plasmin (ibid., page 12108, Table 1).

Biochemistry, 1982, 21, 5891-5897 also discloses monoclonal antibodies to discrete regions of human Glu-plasminogen. This article states that these monoclonal antibodies, i.e., 10-F-1 and 10-V-1, bind to the heavy chain of plasmin (ibid., Abstract).

One known method of assaying human plasminogen is an immunodiffusion method involving the use of an antiserum to human plasminogen. Another method comprises adding an excess of streptokinase to an assay sample to form a complex of it with plasminogen, and determining the plasminogen activity in the sample using a chromogenic synthetic substrate.

The former has the defect that it is extremely difficult to obtain stably an antiserum which has a fixed activity with human plasminogen. The latter is a simple and convenient method, but has slightly low measurement sensivitity. It also has the defect that in assaying hyperbilirubinemic plasma or strongly hemolytic plasma, the assay blank should be corrected.

It is an object of this invention to provide a monoclonal antibody which binds specifically to human plasminogen.

Another object of this invention is to provide a monoclonal antibody which has the ability to bind to human plasminogen but substantially no ability to bind to human plasmin, and thus specifically binds to human plasminogen.

Still another object of this invention is to provide a monoclonal antibody which binds specifically to human plasminogen and permits selective measurement of the amount of plasminogen in a sample, for example, human blood.

Yet another object of this invention is to provide a monoclonal antibody which binds specifically to human plasminogen and is effective for determining the state of clotting and fibrinolysis in vivo or diagnosing diseases such as thrombosis, disseminated intravascular coagulation (DIC).

A further object of this invention is to provide a monoclonal antibody which can be expected to be used for biochemical analysis of proteins, for example for analyzing disorders in protein molecules of plasminogen.

A still further object of this invention is to provide hybridoma cells which can produce the aforesaid monoclonal antibody of the invention.

A yet further object of this invention is to provide a method of producing the monoclonal antibody of the invention by culturing the aforesaid hybridoma cells.

An additional object of this invention is to provide a reagent and a kit for quantifying plasminogen, which comprises the monoclonal antibody of the invention.

Further objects of this invention along with its advantages will become apparent from the following description.

According to this invention, the above objects and advantages of the invention are firstly achieved by a monoclonal antibody characterized by having

(A) the ability to bind to human Glu-plasminogen and human Lys-plasminogen, and

(B) substantially no ability to bind to human Glu-plasmin and human Lys-plasmin.

The monoclonal antibody of this invention is produced by using hybridoma cells.

The hybridoma cells are produced by a technique known as the method of Köhler & Milstein [Köhler & Milstein, Nature 256, 495-497, (1975)]. For example, mice are immunized with human plasminogen, and the spleen cells of the mice are extracted. The mouse spleen cells are fused with mouse myeloma cells. The resulting hybridoma cells are systematically screened for an antibody reactive with human plasminogen which is fixed to microtiter plates, and those hybridoma cells which synthesize and secrete an antibody to human plasminogen are selected. The antibody to human plasminogen which is secreted in the supernatant of the culture of the hybridoma cells is examined for reactivity with plasmin, a plasmin-$\alpha_2$-plasmin inhibitor complex, and a plasmin-$\alpha_2$-macroglobulin complex by the immuno-blotting method. As a result, there are isolated hybridoma cells which show substantially no ability to react with, or bind to, human plasmin but selectively and specifically react with, and bind to, human plasminogen.

The monoclonal antibody of this invention can be isolated from products produced by these hybridoma cells.

The monoclonal antibody so obtained recognizes plasminogen and plasmin distinguishingly, and binds specifically to human plasminogen.

A specific procedure of producing the hybridoma cells of this invention will be described.

A. Isolation and purification of antigen

Human plasminogen used as an antigen is isolated and purified from human plasma by Lysine-Sepharose column chromatography and DEAE Sephadex column chromatography in accordance with the method of Wallen & Wiman.

B. Immunization of mice with human plasminogen

Male Balb/c mice are used, but mice of other strains may also be used. At this time, the immunization should be planned, and the concentration of human plasminogen should be determined, so that sufficient amounts of antigenically stimulated lymphocytes can be formed. For example, the mice are intraperitoneally immunized several times with a small amount of plasminogen at certain intervals, and further it is intravenously administered several times. Several days after the final immunization, the spleen cells are extracted for fusion.

C. Cell fusion

The spleen is aseptically taken out from the immunized animals, and a spleen cell suspension is prepared from it. The spleen cells are fused with mouse myeloma cells from a suitable line by using a suitable fusion promoter. The preferred ratio of the spleen cells to the myeloma cells is from about 20:1 to about 2:1. Use of 0.5 to 1.5 ml of a fusion medium is suitable per about $10^8$ spleen cells. Many myeloma cells suitable for cell fusion are known. In the present invention, P3-X63-Ag8-U1 cells (to be abbreviated as P3-U1) [see D. E. Yelton et al.: Current Topics in Microbiology and Immunology, 81, 1 (1978)] are used. They are an 8-azaguanine resistant cell line. They lack hypoxanthine-guanine phosphoribosyl transferase, and therefore do not survive in HAT medium (containing hypoxanthine, aminopterin and thymidine). Furthermore, this cell line is of a non-secretion type which does not secrete an antibody.

Polyethylene glycol having an average molecular weight of 1,000 to 4,000, for example, may be advantageously used as the fusion promoter. Other fusion promoters known in the art may also be used. In the examples of the present invention, polyethylene glycol having an average molecular weight of 1,540 was used.

D. Selection of fused cells

A mixture of the fused cells, non-fused spleen cells and non-fused myeloma cells is diluted in a separate receptacle (such as a microtiter plate) with a selective medium in which the non-fused myeloma cells cannot survive, and cultured for a sufficient period of time to allow the non-fused cells to die (about 1 week). The culture medium may be one which is resistant to a drug such as 8-azaguanine and in which the non-fused myeloma cells cannot survive, for example the aforesaid HAT medium. In the selective medium, the non-fused myeloma cells die away. Since the non-fused spleen cells are non-tumoral, they die after a certain period of time (about 1 week). On the other hand, the fused cells can survive in the selective medium because they have both the tumor-bearing nature of the parent myeloma cells and the nature of the parent spleen cells.

E. Determination of an antibody to human plasminogen

After the hybridoma cells are detected as stated above, the supernatant of the culture medium is collected and screened for an antibody to human plasminogen by enzyme linked immunosorbent assay (to be referred to as ELISA hereinafter).

F. Selection of hybridoma cells capable of producing an antibody which does not bind to human plasmin but binds specifically to human plasminogen

Human plasmin, a plasmin-$\alpha_2$-plasmin inhibitor complex, a plasmin-$\alpha_2$-macroglobulin complex, and plasminogen are subjected to SDS-polyacrylamide gel electrophoresis, blotted electrically on a nitrocellulose membrane, and reacted with the supernatant of the culture medium of the hybridoma cells obtained by screening in section E above. For detecting an antibody binding to an antigen protein, an enzyme-labelled antibody to mouse Ig is used. In this way, hybridoma cells producing an antibody which selectively recognizes human plasmin and plasminogen and binds specifically to plasminogen are selected.

G. Cloning of the hybridoma cells producing the desired antibody

The hybridoma capable of producing the desired antibody can be cloned in two different ways by a suitable method such as a limiting dilution method. In one way, the hybridoma cells are cultured in a suitable medium for a given period of time, and the monoclonal antibody produced by the hybridoma cells can be obtained from the supernatant of the culture medium. In the other, the hybridoma cells can be intraperitoneally injected into a syngenic or semi-syngenic mouse. After a certain period of time, the monoclonal antibody produced by the hybridoma cells can be obtained from the blood and ascites of the host animal.

H. Culturing of cloned hybridoma cells, isolation and purification of monoclonal antibody, and the bindability or reactivity of the monoclonal antibody

Culturing of the cloned hybridoma cells and isolation and purification of a monoclonal antibody from the culture medium are carried out in accordance with conventional methods.

The ability of the resulting monoclonal antibody of this invention to bind to human plasminogen is not substantially inhibited by epsilon-aminocaproic acid. The resulting monoclonal antibody does not substantially affect the activation of human plasminogen with a plasminogen activator such as urokinase and tissue plasminogen activator (TPA).

The resulting monoclonal antibody neither substantially inhibits the thrombolytic activity of a complex of plasminogen and streptokinase.

Human plasminogen can be quantified by using the monoclonal antibody of the invention in accordance with the sandwich method.

The sandwich method generally denotes a method in which an antibody binding to two different sites of an antigen is prepared and the presence or absence of the antigen or its amount is determined. It is described, for example, in Wide, Radioimmunoassay Methods, 199-206 (1970).

Thus, the present invention provides an immunological reagent for quantifying human plasminogen by the sandwich method, said reagent comprising a primary antibody fixed to an insoluble solid carrier and a secondary labelled antibody, at least one of said antibodies being the aforesaid monoclonal antibody of the invention.

The monoclonal antibody of the invention when used as the primary antibody fixed to an insoluble carrier and the monoclonal antibody of the invention when used as the secondary labelled antibody in the above reagent are both novel, and are provided for the first time by the present invention.

Reagents provided by this invention do no differ in quality, and permit constant quantification of plasminogen in solution (for example, in plasma) with a good accuracy. Since plasminogen is directly measured, the measurement is accurate and rapid without any effect of foreign materials. Accordingly, the present invention provides a reagent and a kit capable of quantifying human plasminogen accurately and rapidly.

The assay reagent and the method of measuring the content of plasminogen in accordance with this invention will be described more specifically.

The monoclonal antibody (primary antibody) to human plasminogen is fixed to a suitable carrier (such as a plastic container). This monoclonal antibody is referred to as the "fixed antibody". The surface of the insoluble carrier is then coated with a suitable substance (such as a bovine serum albumin) to avoid a non-specific binding between the insoluble carrier and the reagent or the assay sample. The insoluble carrier to which the primary antibody is fixed is contacted and reacted with the assay sample at a given temperature for a given period of time. During this time, the fixed antibody (primary antibody) binds to plasminogen in the assay sample. It is then washed with a suitable washing liquid. A solution (e.g., an aqueous soution) of an antibody to human plasminogen (secondary antibody) which is labelled with a suitable labelling substance is contacted with the plasminogen bound to the fixed antibody at a given temperature for a given period of time to induce reaction of the plasminogen with the secondary antibody. After washing with a suitable washing liquid, the amount of the labelling substance labelling the secondary antibody which is present on the insoluble carrier is measured. The amount of plasminogen in the assay sample can be calculated from the amount of the labelling substance.

In order to use the assay reagent of this invention efficiently, easily and conveniently, it may be formed into a kit together with various auxilliary agents. Thus, the present invention provides an assay kit comprising

(a) a primary antibody fixed to an insoluble carrier,
(b) a secondary labelled antibody,
(c) a solubilizing agent for an assay sample (antigen), and
(d) a washing agent,

at least one of said primary and secondary antibodies being the monoclonal antibody of this invention.

The secondary antibody may be labelled with an enzyme, a radioisotope or a fluorescent substance.

When the secondary antibody is labelled, for example, with an enzyme, the kit of this invention may further contain a substrate for measuring enzyme activity and a reaction stopper for deactivating the enzyme activity in order to permit simple and convenient measurement.

The insoluble carrier used in this invention may, for example, be a plastic container, plastic beads, glass beads or metal particles. These insoluble carriers may, for example, be formed of a polymer such as polystyrene, polyethylene, polypropylene, polyesters, polyacrylonitrile, fluorine resins, crosslinked dextran and polysaccharides, paper, glass, metals, agarose and combinations of these. The insoluble carrier may be in the shape of, for example, a tray, a sphere, fibers, a rod, a disc, a container, a cell, or a test tube.

Advantageously, radioactive substances, enzymes and fluorescent substances are advantageously used as the labelling substance. Examples of the radioactive substances are $^{125}I$, $^{131}I$, $^{14}C$ and $^{3}H$. Examples of the enzymes are alkaline phosphatase, peroxidase and beta-D-galactosidase. Examples of the fluorescent substances are fluorescein isothiocyanate and tetramethyl Rhodamine isothiocyanate. These examples are not limitative, and any other labelling substances which can be used in immunological assay methods may be selected in this invention.

The following Examples illustrate the present invention in detail.

EXAMPLE 1

(1) Preparation of human plasminogen

From 200 ml of human plasma, 14 mg of human plasminogen was obtained by using lysine-Sepharose and DEAE-Sephadex.

(2) Immunization of mice

Male Balb/c mice were immunized intraperitoneally with an emulsion of 100 micrograms of human plasminogen and complete Freund's adjuvant twice with an interval of 14 days. Seven days later, 50 micrograms of human plasminogen was additionally administered intravenously together with physiological saline. Four days after the final immunization, the mouse spleen cells were used for cell fusion.

(3) Preparation of a suspension of the spleen cells

The spleen cells were taken out aseptically and passed through a stainless steel mesh to give a suspension of the spleen cells. The suspension was transferred to RPMI-1640 medium (made by GIBCO) supplemented with 0.39 g/liter of L-glutamine, 0.2 g/liter of kanamycin sulfate and 2.0 g/liter of $NaHCO_3$. The cells which proliferated were washed three times with RPMI-1640 and again suspended in RPMI-1640 medium.

(4) Preparation myeloma cells

Mouse myeloma cells P3-UU1 were cultured in RPMI-1640 medium supplemented with 0.39 g/liter of L-glutamine, 0.2 g/liter of kanamycin sulfate, 2.0 g/liter of $NaHCO_3$ and 10% fetal calf serum (this medium is abbreviated as 10% FCS-RPMI-1640). The myeloma cells were in the logarithmic stage of cell division at the time of cell fusion.

(5) Cell fusion

The spleen cells and the myeloma cells were suspended in a ratio of 7:1 in serum-free RPMI-1640 medium, and then centrifuged at about 200G for 5 minutes. The supernatant was removed, and the sedimented matter was incubated at 37°C for 2 minutes together with 1 ml of a 50% solution (pH 8.2) of polyethylene glycol having an average molecular weight of 1,540. Then, 9 ml of serum-free RPMI-1640 was added, and the cells were again suspended carefully for 5 minutes. The suspension was then centrifuged at about 200G for 5 minutes. The cells were again suspended in 10% FCS-RPMI-1640 medium so as to obtain a concentration of $8 \times 10^6$ cells/ml. The suspension was distributed to a 96-well microliter plate (about 100 microliters per well). The fused cells were cultured at 37°C using 5% $CO_2$.

(6) Selection of hybridoma cells capable of producing an antibody to human plasminogen and their culturing

One day after the cell fusion, HAT medium was added in an amount of 100 microliters per well. Thereafter, every two days, half of the medium was replaced by fresh HAT medium, and the fused cells were cultured. Eight days later, the antibodies contained in the supernatant of the culture medium of the hybridoma cells were screened by ELISA. Human plasminogen was used for screening, and an alkaline phosphatase-labelled goat anti-mouse antibody was used as a second antibody.

All 464 wells were positive by ELISA, and produced an antibody to human plasminogen.

When active proliferation of the cells was observed, the HT medium was added. The medium was replaced by fresh HT medium every other day four times in all. Thereafter, the culturing was carried out using ordinary 10% FCS-RPMI-1640 medium.

EXAMPLE 2

Selection of hybridoma cells capable of producing an antibody which binds specifically to human plasminogen but does not bind to human plasmin

The hybridoma cells producing antibodies to human plasminogen were screened from the supernatant of the culture medium by ELSA for hybridoma cells capable of producing an antibody which binds specifically to human plasminogen but does not bind to human plasmin.

Human plasmin, a plasmin-$\alpha_2$-plasmin inhibitor complex and a plasmin-$\alpha_2$-macroglobulin complex were used as antigens in the screening. An alkaline phosphatase-labelled mouse antibody to goat anti-mouse antibody was used as a second antibody.

It was found that out of those wells in which antibodies to human plasminogen were produced, 18 wells bound specifically to human plasminogen without binding to human plasmin, the plasmin-$\alpha_2$-plasmin

inhibitor complex and the plasmin-$\alpha_2$-macroglobulin complex.

EXAMPLE 3

Cloning of the hybridoma cells

The hybridoma cells (3HI) selected by screening in Example 2 were cloned by the following procedure.

The 3HI cells were diluted so that there was 0.9 cell per well of a 96-well microtiter plate. Balb/c mouse thymus cells were added as feeder cells, distributed to the plate, and cultured in 10% FCS-RPMI-1640 medium. The medium was observed under a microscope, and it was determined that single cell colonies formed. The hybridoma cells in the supernatant of the culture medium were screened by enzyme-linked immunosorbent assay for an antibody to human plasminogen.

Twenty-three wells in total were positive by ELISA, and produced a monoclonal antibody which specifically binds to human plasminogen.

Other hybridoma cells were cloned by the same operation as above to obtain a monoclone.

Purification of the monoclonal antibody

In order to produce large amounts of monoclonal antibodies to human plasminogen, about $10^7$ hybridoma cells were intraperitoneally injected into Balb/c mouse pretreated with pristane. About one week later, the antibodies were isolated from the ascites and purified by the method of Ey et al. [P. L. Ey, S. J. Prowse and C. R. Jenkin, Immunochemistry, 15, 429-436 (1978)] using a protein A-Sepharose 4B column. Twenty milligrams of monoclonal antibodies to human plasminogen were obtained from 2.5 ml of the ascites.

Characterization of the purified monoclonal antibodies

The particular classes of the purified monoclonal antibodies were determined by the Ouchterlony gel diffusion test using class-specific antimouse-immunoglobulin antisera. The results are shown in Table 1 below.

Table 1

| Antibody | $IgG_1$ | $IgG_{2b}$ | K |
|---|---|---|---|
| 3H1E6 | + | | + |
| 3H1G5 | + | | + |
| 3H1H6 | + | | + |
| 4A3E9 | | + | + |
| 4A3F7 | | + | + |
| 4A3G1 | | + | + |

In the following Examples, the above two purified monoclonal antibodies 3H1E6 and 4A3E8 were examined in detail for various properties.

EXAMPLE 4

Effect of monoclonal antibodies on the activation of plasminogen with urokinase

Human plasminogen and the monoclonal antibody 3H1E6 or 4A3E8 were mixed in each of the mole ratios indicated in Table 2, and reacted at 37 ° C for 30 minutes. The reaction mixture was left to stand overnight at 4 ° C.

Two hundred units of urokinase were added to the resulting solution and reacted at 37°C for 25 minutes. The reaction mixture was then stirred, and 10 microliters of it was added to the wells of a fibrin plate. After incubation at 37°C for 4 hours, the area of fibrin dissolved was measured. A well to which was added a reaction mixture obtained by activating plasminogen not reacted with the monoclonal antibody with urokinase was used as a control, and the area of fibrin dissolved in this control was taken as 100.

**Table 2**

| Sample | Mole ratio of plasminogen to antibody | Area of fibrin dissolved (%) |
|---|---|---|
| Control | 1:0 | 100 |
| 4A3E8 | 1:0.5<br>1:1<br>1:2 | 102<br>105<br>105 |
| 3H1E6 | 1:0.5<br>1:1<br>1:2 | 99<br>97<br>101 |
| Anti-plasminogen polyclonal antibody | 1:0.5<br>1:1<br>1:2 | 60<br>21<br>12 |

The results shown in Table 2 demonstrate that the anti-plasminogen monoclonal antibodies 4A3E8 and 3H1E6 do not affect the activation of plasminogen with urokinase; namely, plasminogen binding to the antibodies is not inhibited but converted to plasmin by the action of a plasminogen activator such as urokinase.

EXAMPLE 5

Ability of various antibodies to bind to plasminogen and plasmin in the presence of epsilon-aminocaproic acid

The ability of each of the antibodies indicated in Table 3 was examined in the presence of 20 mM epsilon-aminocaproic acid (εACA) by ELISA.

Each of plasminogen and plasmin as an antigen was added to a 96-well microtiter plate in a concentration of 5 micrograms/ml at a rate of 100 microliter per well, and left to stand overnight at 4°C to adsorb it on the plate. A phosphate buffer (to be referred to as PBS hereinafter) containing 20 mM of εACA and 1% BSA was added at a rate of 150 microletres per well. After blocking, the wells were washed three times with PBS containing 0.05% Tween 20. Subsequently, a solution of each of the antibodies diluted to 0.5 micrograms/ml with PBS containing 20 mM εACA was added at a rate of 100 microliters per well and reacted at 37°C for 2 hours. After washing, a solution of an alkaline phosphatase-labelled goat anti-mouse antibody, or a goat anti-rabbit antibody, diluted with PBS containing 20 mM εACA to 3000-fold was added at a rate of 100 microliters per well, and reacted at 37°C for 2 hours. After washing, a solution of an alkaline phosphatase substrate in a concentration of 1 mg/ml was added at a rate of 100 microliters per well. Thirty minutes later, the absorbance of the solution at a wavelength of 405 nm was measured.

As a control, the reaction in the absence of εACA was carried out as above.

The results are summarized in Table 3.

**Table 3**

| Antibody No. | Antigen sample | A405nm | Binding ability |
|---|---|---|---|
| 3H1E6 | εACA + plasminogen | 1.256 | Yes |
| | Plasminogen | 0.781 | Yes |
| | εACA + plasmin | 0.112 | No |
| | Plasmin | 0.115 | No |
| 4A3E8 | εACA + plasminogen | 0.287 | Yes |
| | Plasminogen | 0.234 | Yes |
| | εACA + plasmin | 0.243 | Yes |
| | Plasmin | 0.232 | Yes |
| Rabbit anti-plasminogen polyclonal Ig | εACA + plasminogen | 1.420 | Yes |
| | Plasminogen | 1.595 | Yes |
| | εACA + plasmin | 1.457 | Yes |
| | Plasmin | 1.504 | Yes |
| Anti-$\alpha_2$PI monoclonal Ig 2H12D5 | εACA + plasminogen | 0.117 | No |
| | Plasminogen | 0.114 | No |
| | εACA + plasmin | 0.116 | No |
| | Plasmin | 0.113 | No |

The results demonstrate that the two monoclonal antibodies, 3H1E6 and 4A3E8, react with epsilon-amino-caproic acid and bind also to plasminogen (or plasmin) which has undegone structural change.

EXAMPLE 6

Reactivity of monoclonal antibodies with Glu-plasminogen and Lys-plasminogen (reduced type, non-reduced type), plasmin, and tissue plasminogen activator (t-PA)

Each of the various antigens indicated above (amount of protein 0.5 microgram) was subjected to 10% SDS-polyacrylamide gel electrophoresis, and then the protein was fixed electrically onto a nitrocellulose membrane at 50 V for 1 hour. The nitrocellulose membrane was blocked with Tris buffer containing 3% w/w gelatin (to be referred to as TBS), and reacted overnight at room temperature with a solution of each of monoclonal antibodies, 3H1E6 and 4A3E8 (concentration 1 microgram/ml; diluted with 1% gelatin-TBS). The nitrocellulose membrane was washed three times with TBS containing 0.05% Tween 20, and then a solution of a peroxidase-labelled goat anti-mouse antibody, diluted to 3000-fold with 1% gelatin-TBS. The reaction was carried out at room temperature for 2 hours. The nitrocellulose membrane was washed with 0.05% Tween 20-TBS, and transferred into a solution of a peroxidase substrate. After standing for 5 hours, it was taken out and washed with water. The antigen which reacted with the monoclonal antibody could be observed as a dark blue band.

It was found that the monoclonal antibodies 3H1E6 and 4A3E8 bound to both Glu-plasminogen and Lys-plasminogen but did not bind to reduced plasminogen. The homology of the kringle regions (structures) of the tissue plasminogen activator (t-PA) with the kringle domains of plasminogen was high, but neither of the above monoclonal antibodies reacted with t-PA.

Table 4 summarizes the antigen reactivity and properties of the monoclonal antibodies 3H1E6 and 4A3E8.

## Table 4

| Characterization | Antibody No. | |
|---|---|---|
| | 3H1E6 | 4A3E8 |
| Subclass | $r_1$ | $r_2b$ |
| Ability to bind to Glu-plasminogen | + | + |
| Ability to bind to Lys-plasminogen | + | + |
| Ability to bind to plasmnin | - | + |
| Ability to bind to reduced plasminogen | - | - |
| Effect on activation with urokinase | - | - |
| Ability to bind to ε-ACA-treated plasminogen | + | + |

EXAMPLE 7

Plasminogen and monoclonal antibody 3H1E6 or a rabbit polyclonal antibody were mixed in a suitable mole ratio, and reacted at 37°C for 30 minutes. Streptokinase in an excessive amount with respect to plasminogen was added, and reacted at 37°C for 10 minutes. Thereafter, a substrate solution (S-2251) was added, and reacted at 37°C for 5 minutes. The absorbance of the solution at 405 nm was measured. The results of the measurement are shown in Figure 1 in which the absorbance obtained when human plasminogen and streptokinase were reacted without the addition of the monoclonal antibody is expressed as 100%.

The polyclonal antibody, as a result of binding to plasminogen, inhibited the activation of plasminogen with streptokinase. In contrast, the monoclonal antibody 3H1E6 did not affect the activation.

EXAMPLE 8

Preparation of a calibration curve

Monoclonal antibody "3H1E6" shown in Table 1 in Example 3 was fixed to an insoluble carrier (microtiter plate) as described below.

This monoclonal antibody does not at all bind to free plasmin, plasmin-$\alpha_2$ PI complex and plasmin-$\alpha_2$M complex, but strongly binds specifically to plasminogen.

An antibody component was purified from anti-sera to human plasminogen which was obtained by immunizing rabbit with human plasminogen. The antibody was labelled with peroxidase, and used as a second antibody.

The monoclonal antibody 3H1E6 (20 micrograms/ml) specifically recognizing human plasminogen was left to stand overnight at 4°C on a microtiter plate to fix it. A buffer (20 mM phosphate buffer, 0.135 M NaCl) containing 1% bovine serum albumin was added, and left to stand at room temperature for 4 hours. The plate was washed three times with a washing liquor (20 mM phosphate buffer, 0.135 M NaCl, 0.05% Tween 20) containing 1% bovine serum albumin. Purified human plasminogen, diluted to various concentrations with a diluting solution (20 mM phosphate buffer, 0.135 M NaCl), was added, and reacted at 37°C for 2 hours.

The plate was then washed three times with the same washing liquor as described above, and the peroxidase-labelled second antibody to human plasminogen was added in a concentration of 350 ng/ml, and reacted at 37°C for 1 hour. After washing with the same washing liquor as described above, a substrate (ABTS) was added in a concentration of 0.5 mg/ml. Changes in absorbance at a wavelength of 415 nm per minute were measured by means of ELISA ANALYZER (ETY-96, made by Toyo Measuring Instrument Co., Ltd.). The results were plotted in Figure 2. It is seen from this figure that the concentration of human plasminogen and the changes in absorbance are in a linear relationship.

Accordingly, the amount of plasminogen can be easily determined by using a monoclonal antibody specifically recognizing and binding to human plasminogen as one antibody in the sandwich method.

EXAMPLE 9

Quantification of plasminogen in human plasma

Human plasma was diluted to 400-fold with a phosphate buffer (20 mM phosphate buffer, 0.135 M NaCl, pH 7.2) and used as an antigen. The amount of plasminogen was determined by the sandwich method described in Example 8 using the calibration curve shown in Figure 2.

The results are shown in Table 5 and Figure 3. In Table 5 and Figure 3, the amount of plasminogen is expressed in % by taking the amount of plasminogen in a plasma sample taken from a healthy person as 100%.

The level of plasminogen in blood decreased markedly in DIC and hepatic disease. The measuring kit in accordance with this invention would be effective for diagnosing these diseases. It is also very useful for monitoring in thrombolytic therapy by administration of urokinase, evaluating the efficacy of a drug, and analyzing a diseased condition.

Table 5

| Assay sample | Amount of plasminogen (%) | Case |
|---|---|---|
| 1 | 5 | DIC |
| 2 | 21 | DIC |
| 3 | 10 | DIC |
| 4 | 39 | DIC |
| 5 | 47 | DIC |
| 6 | 83 | hepatic disease |
| 7 | 72 | hepatic disease |

EXAMPLE 10

Hybridoma cells having the property of producing monoclonal antibody 3H1E6 were cultured in RPMI 1640 medium containing 10% fetal calf serum (FCS). After culturing for 2 days at 37°C in the presence of 5% $CO_2$ when the proliferation of the cells was confluent, the supernatant of the culture medium was collected, and centrifuged at 3,000 rpm for 10 minutes. 250 ml of the supernatant was applied to 5 ml of protein A-Sepharose to adsorb the monoclonal antibody in the supernatant. The gel was washed thoroughly with 400 ml of 0.1M phosphate buffer (pH 8.0), and eluted with 0.1M citrate buffer (pH 3.0). Immediately, the eluates were neutralized with 0.1M Tris solution (pH 11.0) and dialyzed against 10 mM Tris-HCl buffer (pH 7.4). There was obtained 4.2 mg of the monoclonal antibody. The properties of the monoclonal antibody were examined, and it was found to be 3H1E6 obtained in Example 3.

**Claims**

**1.** A monoclonal antibody characterized by having
(A) the ability to bind to human Glu-plasminogen and human Lys-plasminogen, and
(B) substantially no ability to bind to human Glu-plasmin and human Lys-plasmin.

**2.** The monoclonal antibody of claim 1 of which ability to bind to human Glu-plasminogen and human Lys-plasminogen is not substantially inhibited by epsilon-aminocaproic acid.

**3.** The monoclonal antibody of claim 1 which does not substantially affect activation of human Glu-plasminogen and human Lys-plasminogen with a plasminogen activator.

**4.** The monoclonal antibody of claim 1 which does not substantially inhibit the thrombolytic activity of a streptokinase-human Glu-plasminogen complex and a streptokinase-human Lys-plasminogen complex.

**5.** The monoclonal antibody of claim 1 which has substantially no ability to bind to a human Glu-plasmin-$\alpha_2$-plasmininhibitor complex and a human Lys-plasmin-$\alpha_2$-plasmin inhibitor complex.

**6.** The monoclonal antibody of claim 1 which has substantially no ability to bind to a human Glu-plasmin-$\alpha_2$-macroglobulin complex and a human Lys-plasmin-$\alpha_2$-macroglobulin complex.

**7.** Hybridoma cells which produce the monoclonal antibody of claim 1.

**8.** The hybridoma cells of claim 7 which are obtained by fusing spleen cells of a mammal immunized with human Glu-plasminogen or human Lys-plasminogen, with myeloma cells.

**9.** The hybridoma cells of claim 8 wherein the mammal is a mouse.

**10.** A method for producing the monoclonal antibody of claim 1, which comprises culturing the hybridoma cells of claim 7, separating the monoclonal antibody produced by the hybridoma cells from other products, and purifying it.

**11.** The monoclonal antibody of claim 1 which is labelled.

**12.** The monoclonal antibody of claim 11 which is labelled with an enzyme, a radioisotope or a fluorescent substance.

**13.** The monoclonal antibody of claim 1 which is fixed to an insoluble carrier.

**14.** The antibody of claim 13 wherein the insoluble carrier is a plastic container, plastic beads, glass beads or metal particles.

**15.** An immunological reagent for quantifying human plasminogen by the sandwich method, comprising a primary antibody fixed to an insoluble carrier and a secondary labelled antibody, at least one of said antibodies being the monoclonal antibody of claim 1.

**16.** The reagent of claim 15 wherein the insoluble carrier is a plastic container, plastic beads, glass beads or metal particles

**17.** An immunological assay kit comprising
(a) a primary antibody fixed to an insoluble carrier,
(b) a secondary labelled antibody,
(c) a dissolving agent for an antigen, and
(d) a washing agent,
at least one of said primary and secondary antibodies being the monoclonal antibody of claim 1.

**18.** The kit of claim 17 in which the secondary labelled antibody is an antibody labelled with an enzyme and which further comprises a substrate for measuring enzyme activity and a reaction stopper for deactivating enzyme activity.

## Patentansprüche

**1.** Monoklonaler Antikörper, dadurch **gekennzeichnet,** daß er
(A) die Fähigkeit, humanes Glu-Plasminogen und humanes Lys-Plasminogen zu binden, besitzt und
(B) im wesentlichen keine Fähigkeit besitzt, humanes Glu-Plasmin und humanes Lys-Plasmin zu binden.

**2.** Monoklonaler Antikörper nach Anspruch 1, dadurch **gekennzeichnet,** daß die Fähigkeit, humanes Glu-Plasminogen und humanes Lys-Plasminogen zu binden, im wesentlichen nicht durch $\epsilon$-Aminocapronsäure inhibiert wird.

**3.** Monoklonaler Antikörper nach Anspruch 1, dadurch **gekennzeichnet,** daß er die Aktivierung von humanem Glu-Plasminogen und humanem Lys-Plasminogen mit einem Plasminogenaktivator im wesentlichen nicht beeinflußt.

**4.** Monoklonaler Antikörper nach Anspruch 1, dadurch **gekennzeichnet,** daß er die thrombolytische Aktivität von einem Streptokinase-humanem Glu-Plasminogenkomplex und einem Streptokinase-humanem Lys-Plasminogenkomplex im wesentlichen nicht inhibiert.

5. Monoklonaler Antikörper nach Anspruch 1, dadurch **gekennzeichnet,** daß er im wesentlichen keine Bindungsfähigkeit an einen humanen Glu-Plasmin-$\alpha_2$-Plasmin-Inhibitorkomplex und an einen humanen Lys-Plasmin-$\alpha_2$-Plasmin-Inhibitorkomplex aufweist.

6. Monoklonaler Antikörper nach Anspruch 1, dadurch **gekennzeichnet,** daß er im wesentlichen keine Bindungsfähigkeit an einen humanen Glu-Plasmin-$\alpha_2$-Makroglobulinkomplex und an einen humanen Lys-Plasmin-$\alpha_2$-Makroglobulinkomplex aufweist.

7. Hybridomazellen, dadurch **gekennzeichnet,** daß sie den monoklonalen Antikörper nach Anspruch 1 erzeugen.

8. Hybridomazellen nach Anspruch 7, dadurch **gekennzeichnet,** daß sie durch Fusionierung von Milzzellen eines Säugers, der mit humanem Glu-Plasminogen oder humanem Lys-Plasminogen immunisiert ist, mit Myelomazellen erhalten worden sind.

9. Hybridomazellen nach Anspruch 8, dadurch **gekennzeichnet,** daß der Säuger eine Maus ist.

10. Verfahren zur Herstellung des monoklonalen Antikörpers nach Anspruch 1, dadurch **gekennzeichnet,** daß die Hybridomazellen von Anspruch 7 gezüchtet werden, der durch die Hybridomazellen erzeugte monoklonale Antikörper von anderen Produkten abgetrennt wird und gereinigt wird.

11. Monoklonaler Antikörper nach Anspruch 1, dadurch **gekennzeichnet,** daß er markiert ist.

12. Monoklonaler Antikörper nach Anspruch 11, dadurch **gekennzeichnet,** daß er mit einem Enzym, einem Radioisotop oder einer fluoreszierenden Substanz markiert ist.

13. Monoklonaler Antikörper nach Anspruch 1, dadurch **gekennzeichnet,** daß er an einen unlöslichen Träger fixiert ist.

14. Monoklonaler Antikörper nach Anspruch 13, dadurch **gekennzeichnet,** daß der unlösliche Träger ein Kunststoffbehälter, Kunststoffkügelchen, Glaskügelchen oder Metallteilchen ist.

15. Immunologisches Reagens für die quantitative Bestimmung von humanem Plasminogen nach dem Sandwichverfahren, dadurch **gekennzeichnet,** daß es einen an einen unlöslichen Träger fixierten primären Antikörper und einen sekundären markierten Antikörper umfaßt, wobei mindestens einer der Antikörper der monoklonale Antikörper nach Anspruch 1 ist.

16. Reagens nach Anspruch 15, dadurch **gekennzeichnet,** daß der unlösliche Träger ein Kunststoffbehälter, Kunststoffkügelchen, Glaskügelchen oder Metallteilchen ist.

17. Immunologisches Analysekit, dadurch **gekennzeichnet,** daß es
    (a) einen an einen unlöslichen Träger fixierten primären Antikörper,
    (b) einen sekundären markierten Antikörper,
    (c) ein Auflösungsmittel für ein Antigen und
    (d) ein Waschmittel
    umfaßt, wobei mindestens einer der primären und sekundären Antikörper der monoklonale Antikörper von Anspruch 1 ist.

18. Kit nach Anspruch 17, dadurch **gekennzeichnet,** daß der sekundäre markierte Antikörper ein Antikörper ist, der mit einem Enzym markiert ist, und daß es weiter ein Substrat für die Messung der Enzymaktivität und ein Reaktions-Beendigungsmittel für die Deaktivierung der Enzymaktivität umfaßt.

## Revendications

1. Anticorps monoclonal caractérisé en ce que :
    (A) il a l'aptitude à se lier au Glu-plasminogène humain et au Lys-plasminogène humain, et
    (B) il n'a substantiellement aucune aptitude à se lier à la Glu-plasmine humaine et à la Lys-plasmine humaine.

2. Anticorps monoclonal selon la revendication 1, dont l'aptitude à se lier au Glu-plasminogène humain et au Lys-plasminogène humain n'est pas inhibée de façon substantielle par l'acide epsilon-aminocaproïque.

3. Anticorps monoclonal selon la revendication 1, qui n'affecte pas de façon substantielle l'activation du Glu-plasminogène humain et du Lys-plasminogène humain par un activateur de plasminogène.

4. Anticorps monoclonal selon la revendication 1, qui n'inhibe pas de façon substantielle l'activité thrombolytique d'un complexe streptokinase-Glu-plasminogène humain, et d'un complexe streptokinase -Lys-plasminogène humain.

5. Anticorps monoclonal selon la revendication 1, qui n'a substantiellement aucune aptitude à se lier à un complexe inhibiteur Glu-plasmine humaine-$\alpha_2$-plasmine, et à un complexe inhibiteur Lys-plasmine humaine-$\alpha_2$-plasmine.

6. Anticorps monoclonal selon la revendication 1, qui n'a substantiellement aucune aptitude à se lier à un complexe Glu-plasmine humaine-$\alpha_2$-macroglobuline et à un complexe Lys-plasmine humaine-$\alpha_2$- macroglobuline.

7. Cellules d'hybridome qui produisent l'anticorps monoclonal de la revendication 1.

8. Cellules d'hybridome selon la revendication 7, qui sont obtenues par fusion de cellules de rate d'un mammifère, immunisé avec du Glu-plasminogène humain ou du Lys-plasminogène humain, avec des cellules de myélome.

9. Cellules d'hybridome selon la revendication 8, où le mammifère est une souris.

10. Procédé pour produire l'anticorps monoclonal de la revendication 1, qui comporte le fait de cultiver les cellules d'hybridome de la revendication 7, la séparation de l'anticorps monoclonal produit par les cellules d'hybridome, à partir d'autres produits, et sa purification.

11. Anticorps monoclonal selon la revendication 1, qui est marqué.

12. Anticorps monoclonal selon la revendication 11, qui est marqué par une enzyme, un radioisotope ou une substance fluorescente.

13. Anticorps monoclonal selon la revendication 1, qui est fixé à un support insoluble.

14. Anticorps selon la revendication 13, où le support insoluble est un récipient plastique, des billes de plastique, des billes de verre ou des particules de métaux.

15. Réactif immunologique pour déterminer de façon quantitative le plasminogène humain par la méthode sandwich, comportant un anticorps primaire fixé à un support insoluble, et un anticorps secondaire marqué, au moins un de ces anticorps étant l'anticorps monoclonal de la revendication 1.

16. Réactif selon la revendication 15, où le support insoluble est un récipient plastique, des billes plastiques, des billes de verre ou des particules métalliques.

17. Trousse d'essai immunologique comportant
    (a) un anticorps primaire fixé à un support insoluble,
    (b) un anticorps secondaire marqué,
    (c) un agent pour dissoudre l'antigène, et
    (d) un agent de lavage,

    au moins un desdits anticorps primaire et secondaire étant l'anticorps monoclonal de la revendication 1.

18. Trousse selon la revendication 17, dans laquelle l'anticorps secondaire marqué est un anticorps marqué avec une enzyme, et qui comporte en outre un substrat pour mesurer l'activité de l'enzyme et un bloqueur de réaction pour désactiver l'activité de l'enzyme.

FIG. 1

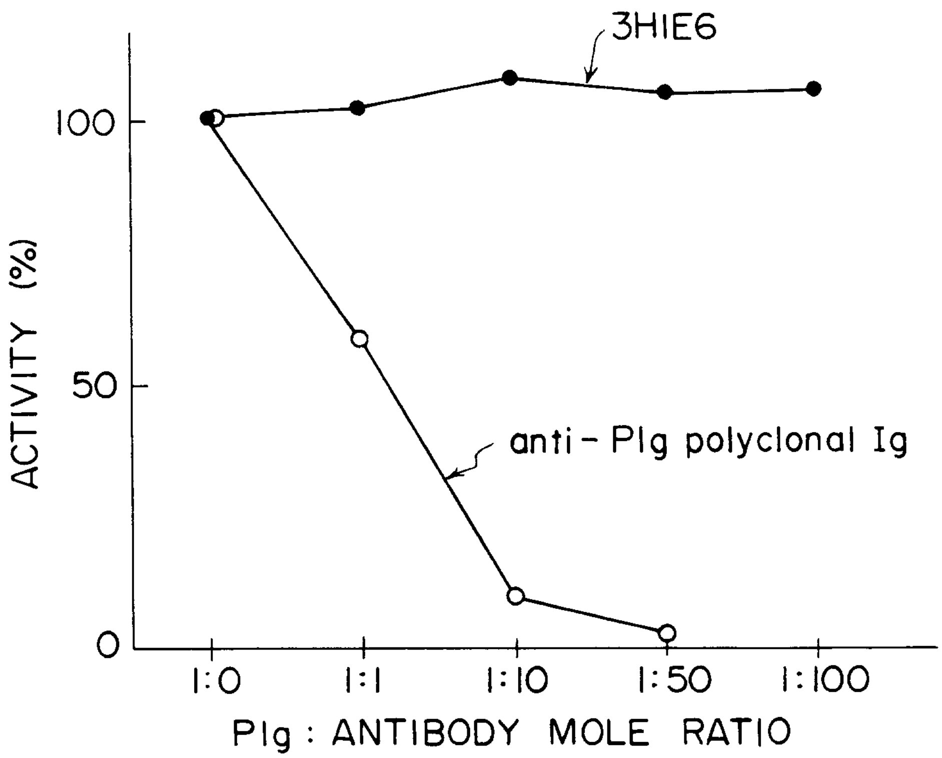
3HIE6
100
50
0
ACTIVITY (%)
anti-Plg polyclonal Ig
1:0
1:1
1:10
1:50
1:100
Plg : ANTIBODY MOLE RATIO

FIG. 3

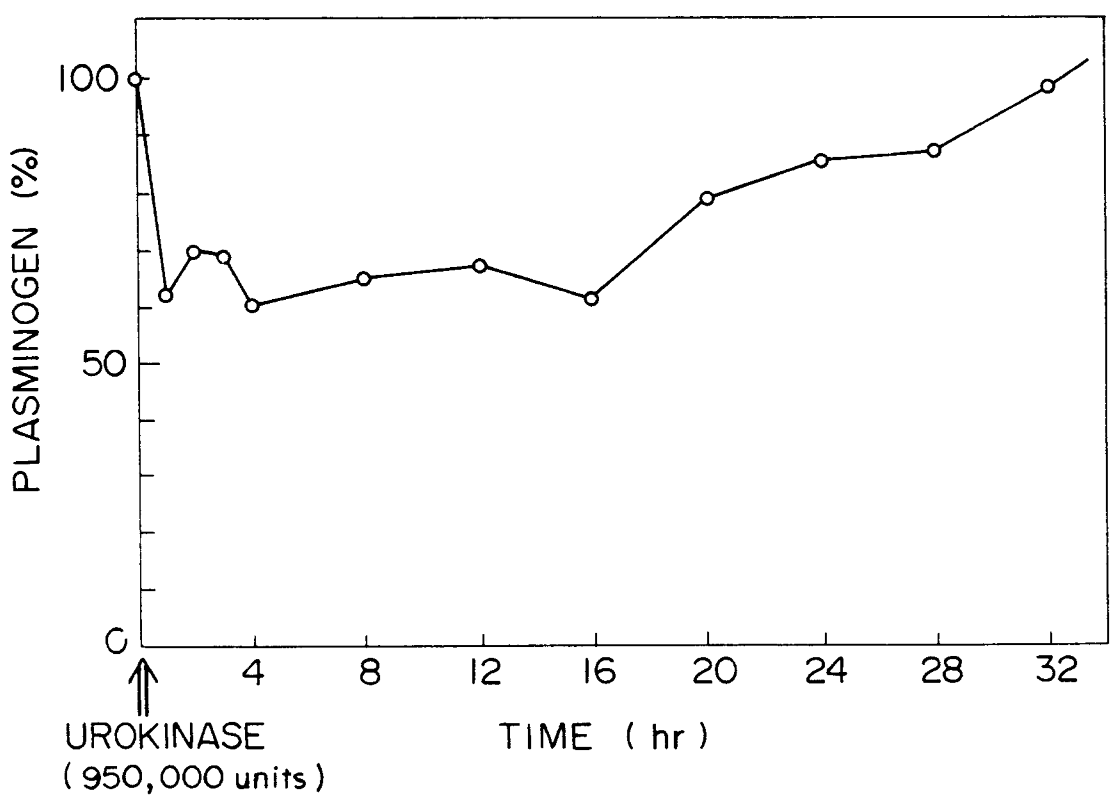
100
50
0
PLASMINOGEN (%)
4
8
12
16
20
24
28
32
UROKINASE
(950,000 units)
TIME (hr)

FIG. 2

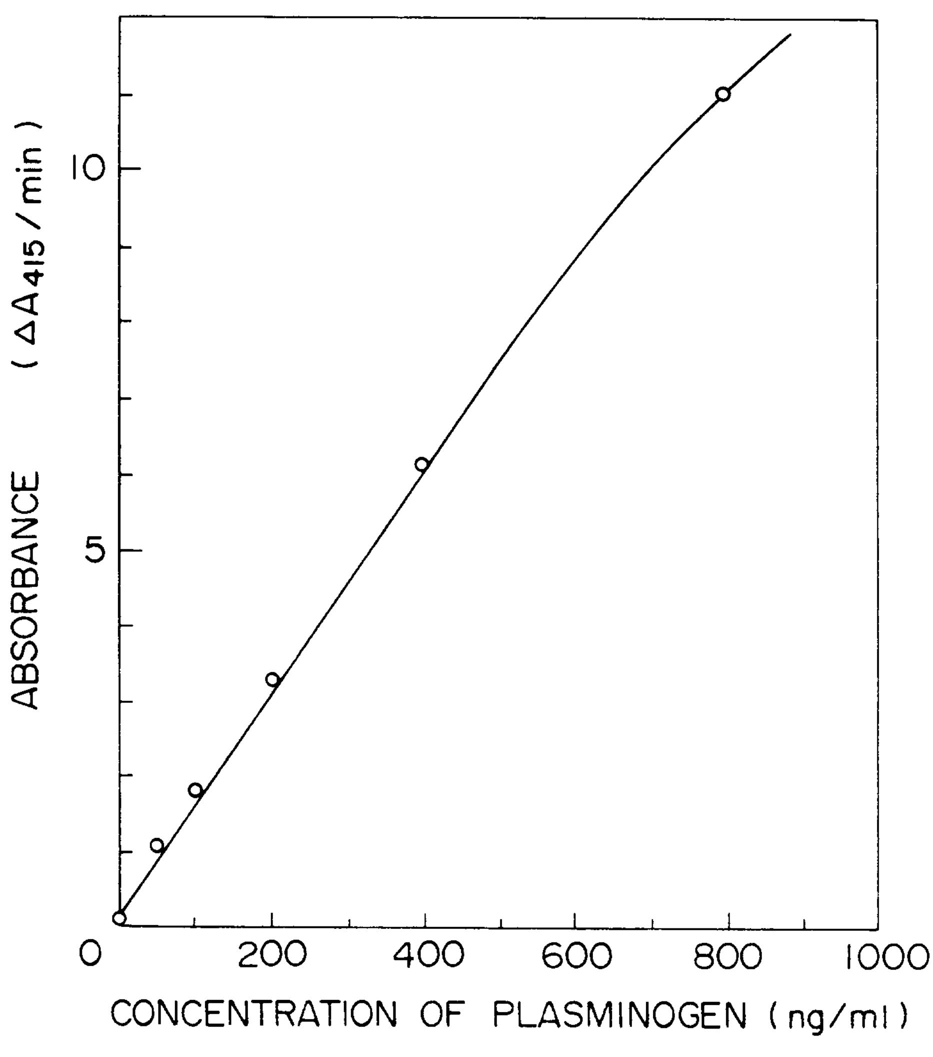
ABSORBANCE ( ΔA415 / min )
10
5
0
200
400
600
800
1000
CONCENTRATION OF PLASMINOGEN ( ng/ml )